Europäisches Patentamt

(19) European Patent Office

Office européen des brevets

(11) Numéro de publication: **0 036 339**
**B1**

(12) **FASCICULE DE BREVET EUROPEEN**

(45) Date de publication du fascicule du brevet: **21.12.83**

(51) Int. Cl.³: **A 61 L 9/14, B 05 B 7/30**

(21) Numéro de dépôt: **81400039.4**

(22) Date de dépôt: **13.01.81**

(54) Procédé de désinfection d'un local et dispositif de traitement pour la mise en oeuvre du procédé.

(30) Priorité: **14.01.80 FR 8001134**

(43) Date de publication de la demande:
**23.09.81 Bulletin 81/38**

(45) Mention de la délivrance du brevet:
**21.12.83 Bulletin 83/51**

(84) Etats contractants désignés:
**AT BE CH DE GB IT LI LU NL SE**

(56) Documents cités:
**FR - A - 940 569**
**FR - A - 964 722**
**FR - A - 991 752**
**FR - A - 1 047 514**
**FR - A - 1 568 295**
**FR - A - 2 026 337**

**W.B. HUGO: "Inhibition and destruction of the microbial cell", pages 593-595, publié 1971, Academic Press, London, GB**

(73) Titulaire: **Laboratoires ANIOS, SARL dite**
**46, rue de Soubise**
**F-59100 Roubaix (Nord) (FR)**

(72) Inventeur: **Letartre, Thiéry**
**6, Allée de la Cerisaie**
**Marcq-En-Baroeul (Nord) (FR)**
Inventeur: **Lepage, Christian**
**1-17, Rue Jules Vallès**
**Loos (Nord) (FR)**
Inventeur: **Decotignies, Vincent**
**111, rue de Lille**
**Roubaix (Nord) (FR)**

(74) Mandataire: **Lepage, Jean-Pierre**
**c/o BUGNION 23/25, rue N. Leblanc**
**F-59011 Lille Cedex - (Nord) (FR)**

Courier Press, Leamington Spa, England.

Procédé de désinfection d'un local et dispositif de traitement pour la mise en oeuvre du procédé

L'invention est relative à un procédé de désinfection d'un local, tel que par exemple, un salle ou un bloc opératoire. L'invention est également relative à un dispositif de traitement, mettant en oeuvre le procédé.

La désinfection d'un local est réalisée soit au moyen d'un gaz désinfectant, soit au moyen d'un micro-brouillard désinfectant.

Des procédés de désinfection actuellement existants consistent à saturer l'atmosphère d'un local au moyen d'un gaz désinfectant.

D'autres procédés actuellement existants consistent à pulvériser dans l'atmosphère du local un micro-brouillard d'une solution désinfectante. En particulier, le brevet FR—A—991.752 décrit un procédé et un dispositif de pulvérisation d'un liquide utilisé pour la désinfection des locaux. Ce procédé consiste à pulvériser le liquide sous forme d'un micro-brouillard homogène qui est maintenu à l'état de suspension pour l'empêcher de se déposer. Pour cela, on amène le liquide désinfectant sur une surface de distribution rotative, sous la forme d'une fine pellicule que l'on projette sur une surface de pulvérisation.

C'est au second type de procédè que se rapporte la présente invention.

Les procédés actuels de ce type présentent deux inconvénients. D'une part, il est nécessaire de pulvériser la solution désinfectante sous une forme de micro-gouttes de dimension la plus réduite possible, de manière à augmenter la surface de contact entre l'atmosphère et la solution désinfectante. D'autre part, il est nécessaire de pulvériser en excès la solution désinfectante, et après la désinfection, un taux résiduel non nul de solution désinfectante subsiste dans l'atmosphère.

Certaines solutions désinfectantes couramment utilisées présentent une odeur forte qu'il est nécessaire d'éliminer de manière à rendre le local de nouveau opérationnel.

Il est alors possible d'aérer le local, de manière à en renouveler l'atmosphère, ce qui offre la possibilité d'une recontamination. Il est également possible de pulvériser dans l'atmosphère une autre solution néutralisant la solution désinfectante. Cette dernière doit cependant être dosée avec précision. En effet, si elle est pulvérisée par excès, c'est sa propre odeur qui risque de se substituer à l'odeur de la solution désinfectante résiduelle. Par contre, si elle est pulvérisée de manière insuffisante, l'odeur de la solution désinfectante subsiste.

Par ailleurs, il faut remarquer que le produit de la réaction de la solution de désinfection sur la solution de neutralisation doit être lui aussi éliminé, ce qui nécessite un nettoyage du local en particulier lorsqu'il se présente sous forme solide, et par exemple de poudre.

Ainsi, les procédés actuels de désinfection exigent un temps d'immobilisation des locaux particulièrement long.

Un des buts de la présente invention est de proposer un procédé de désinfection d'un local qui permette de rendre ce local de nouveau opérationnel après un temps plus court que les procédés actuellement existants, la désinfection, dans le cas présent, consistant à désinfecter effectivement l'atmosphère, c'est-à-dire présenter une action bactéricide, fongicide, sporicide, et virulucide sur celle-ci, et à éliminer le taux résiduel de la solution désinfectante présent dans l'atmosphère en évitant la recontamination de celle-ci, qui pourrait être provoquée par une ventilation, et en évitant la nécessité d'un nettoyage en fin d'opération, qui pourrait être dû à la pulvérisation d'une solution neutralisant le désinfectant.

Un autre but de la présente invention est de proposer un dispositif de traitement de l'atmosphère qui permette d'éliminer efficacement le taux résiduel de la solution désinfectante présent dans l'atmosphère après la désinfection.

D'autres buts et avantages de la présente invention apparaîtront au cours de la description qui va suivre.

Le procédé de désinfection d'un local tel que par exemple une salle ou un bloc opératoire, dans lequel on pulvérise dans l'atmosphère un micro-brouillard d'une solution d'aldéhyde formique pendant une durée determinée, est caractérisé par le fait qu'ensuite on provoque le passage de l'atmosphère pendant une durée déterminée, afin d'éliminer le taux résiduel de la solution d'aldéhyde formique présent dans l'atmosphère désinfectée, dans une enceinte à l'intérieur de laquelle on réalise un micro-brouillard d'une solution aqueuse neutralisant l'aldéhyde formique véhiculé par l'atmosphère, que l'on sépare au niveau de la sortie de l'enceinte les particules solides et liquides de l'atmosphère gazeuse débarrassée de l'aldéhyde formique.

Le dispositif de traitement de l'atmosphère désinfectée est caractérisé par le fait que sa sortie est constituée par une cheminée sensiblement cylindrique de révolution, munie d'une pluralité de portions de disques s'étendant depuis la périphérie vers la zone centrale de celle-ci, sensiblement perpendiculairement à son axe, réparties à différents niveaux et disposées de manière à obturer au moins une fois l'orifice de sortie de la cheminée lorsqu'ils sont vus selon son axe.

L'invention sera mieux comprise si l'on se réfère à la description ci-dessous, ainsi qu'aux dessins en annexe qui en font partie intégrante.

La figure 1 schématise vu de côté un générateur d'aérosol utilisé dans le procédé selon l'invention.

La figure 2 est une vue de dessus de générateur représenté en figure 1.

La figure 3 schématise vu de côté un dispositif de traitement.

Les figures 4a à 4h représentent respectivement la position relative des différentes portions de disques du dispositif de la figure 3.

Selon la présente invention, on pulvérise dans l'atmosphère du local que l'on désire désinfecter un micro-brouillard d'une solution désinfectante. Cette solution est constituée par de l'aldéhyde formique, également connu sous le nom de formol, et d'un solvant. Le formol est un produit connu pour ses qualités de désinfection. Le solvant, par ailleurs, permet de réguler l'humidité de l'atmosphère du local.

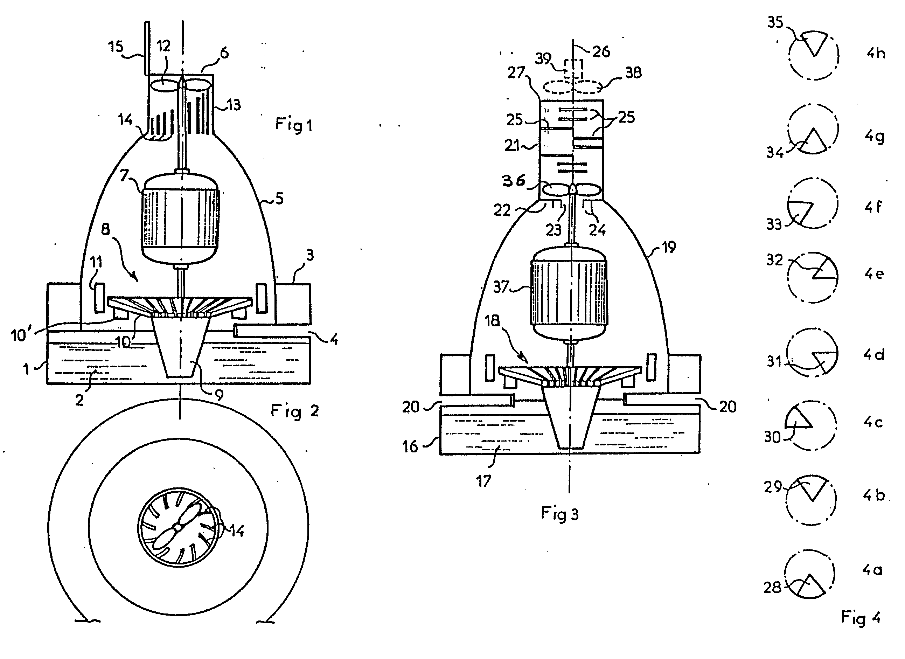

Un dispositif tel que celui schématisé dans les figures 1 et 2 permet de pulvériser dans l'atmosphère du local un micro-brouillard de solution désinfectante dont la dimension des particules est inférieure à 5 microns. Il est connu que pour une solution désinfectante telle qu'une solution à base de formol, plus la dimension des particules est fine, plus le désinfectant est agressif.

Le générateur d'aérosol comprend tout d'abord un réservoir 1 à l'intérieur duquel se trouve la solution désinfectante 2. Ce réservoir présente une forme sensiblement cylindrique de révolution.

Dans sa partie supérieure, un carter 3 recouvre le réservoir 1, et sur une partie de la circonférence de jonction entre le réservoir 1 et le carter 3 se trouve une ouverture 4 de communication avec l'atmosphère extérieure. Le carter 3 est prolongé par un carter principal 5 qui présente approximativement une forme ovoïde renversée. Ce carter protège la partie mécanique du générateur d'aérosol, et canalise le micro-brouillard vers la sortie 6 du générateur.

Le micro-brouillard est réalisé par des moyens d'un type connu qui ont été schématisés en 8. Ils comprennent, par exemple, une crépine 9 de forme tronconique inversée, et plogeant partiellement dans la solution désinfectante à pulvériser. Cette crépine est creuse, et sa partie inférieure débouche dans la partie centrale d'un plateau de pulvérisation 10. La crépine 9 et le plateau 10 sont entraînés par un moteur 7. Sous l'effet de la rotation, la solution désinfectante est aspirée à l'intérieur de la crépine puis projetée par le plateau 10 contre une couronne fixe 11 qui est percée d'ouvertures. Par ailleurs, sous le plateau 10, des pales 10' aussurent le brassage de l'atmosphère extérieure introduite par l'ouverture 4, et favorisent le mélange du micro-brouillard et de l'atmosphère qui le véhicule.

L'atmosphère chargée des particules de micro-brouillard est par ailleurs chassée de générateur par une hélice de ventilateur 12 entraînée en rotation par le moteur 7, et située au niveau de la sortie 6 du générateur.

Les moyens de pulvérisation 8 créent en fait un micro-brouillard dont les particules présentent des dimensions variables. Le générateur d'aérosol schématisé aux figures 1 et 2 est muni de moyens qui retiennent les particules de dimensions les plus importantes, et qui ne laissent passer que les très fines particules, d'une dimension sensiblement inférieure à 5 microns.

Ces moyens sont constitués par une cheminée 13, sensiblement cylindrique de révolution, qui prolonge de manière continue le carter principal 5. Une pluralité d'ailettes 14, orientées sensiblement parallèlement à l'axe de la cheminée, s'étendent depuis la périphérie interne de celle-ci vers sa partie centrale.

Ainsi que le montre la figure 1, la hauteur des ailettes varie d'une manière sensiblement continue sur chaque demi-circonférence de la cheminée, et leur extrémité inférieure se situe dans un plan sensiblement perpendiculaire à l'axe de la cheminée.

De préférence, ainsi que cela est visible en figure 2, les ailettes 14 s'étendent depuis la périphérie interne de la cheminée vers sa partie centrale selon une courbe douce.

De préférence, l'hélice 12 du ventilateur est placée au-dessus des ailettes 14.

Les particules de la solution désinfectante d'une dimension importante seront piégées par ces ailettes 14 et recyclées vers le réservoir 1 en s'écoulant le long du carter principal 5. Ainsi, seules les particules d'une dimension approximativement inférieure à 5 microns seront pulvérisées dans le local.

Par ailleurs, dans un mode préférentiel de réalisation, le générateur d'aérosol présente un couvercle basculant 15 susceptible d'obturer la partie supérieure de la cheminée 13 lorsque le générateur n'est pas en service. Ce couvercle évite alors l'évaporation de la solution désinfectante qui répandrait dans l'atmosphère une odeur désagréable.

Le générateur d'aérosol peut présenter par ailleurs à l'intérieur du carter principal 5 d'autres parois, en particulier favorisant la circulation du micro-brouillard, et protégeant le moteur électrique 7 de celui-ci. Ces parois sont à la portée de l'Homme de l'Art, et n'ont pas été représentées en figure 1.

Selon l'invention, la pulvérisation du micro-brouillard dans l'atmosphère du local est réalisée pendant une durée déterminée, et par exemple de 2 à 4 heures, en fonction du volume du local.

Durant ce temps, la solution désinfectante, c'est-à-dire le formol, réagit sur les bactéries, les virus, contenus dans l'atmosphère du local.

Après cette durée, un résidu de formol subsiste dans l'atmosphère du local. Le formol se trouve alors à l'état gazeux, et répand une odeur forte dans le local.

La seconde phase du procédé de désinfection selon l'invention se propose d'éliminer l'odeur du formol en éliminant le formol lui-même.

Ce produit se caractérise par une forte solubilité. La seconde phase du procédé consiste donc à piéger le formol, c'est-à-dire à le dissoudre puis à le soumettre à une réaction chimique dénaturante qui l'élimine.

Ces réactions chimiques sont réalisées à l'intérieur d'une enceinte. Ainsi, l'atmosphère n'est pas soumise à une nouvelle pulvérisation d'une solution neutralisant le formol.

A l'intérieur de cette enceinte, la solution de neutralisation est pulvérisée sous la forme d'un micro-brouillard, dont les particules présentent toutefois une dimension plus importante que le micro-brouillard de désinfection.

On provoque une circulation forcée de l'atmosphère du local à l'intérieur de l'enceinte, de manière à la mettre en contact avec les particules du micro-brouillard. La solution de neutralisation réagit alors sur le formol. Après la réaction, l'atmosphère débarrassée du formol est de nouveau évacuée vers l'extérieur, et les particules liquides et solides qu'elle véhicule, et qui sont constituées par la pulvérisation de la solution de neutralisation, et par les produits de cette réaction sont piégés à l'intérieur de l'enceinte, et ne sont donc pas recyclés dans l'atmosphère du local.

De préférence, la solution de neutralisation est à base de sulfite, et plus particulièrement de sulfite de sodium dilué dans de l'eau déminéralisée. Par ailleurs, d'autres additifs tels qu'un tensioactif peuvent être ajoutés.

D'autres substances pourraient être utilisées pour neutraliser le formol. Cependant, le sulfite de sodium est préféré, car il permet d'obtenir un composé stable avec un rendement maximum, et une vitesse de réaction rapide même à froid, c'est-à-dire à température ambiante.

Il faut par ailleurs remarquer que la dissolution du sulfite de sodium dans l'eau entraîne la formation d'ions $OH^-$, et que donc la solution de neutralisation présente un caractère basique. Ainsi, on ajoute un acide, et de préférence de l'acide chlorhydrique à la solution de neutralisation pour maintenir le pH de la solution voisin de 7, et éviter ainsi l'attaque chimique du matériel et son entartrage.

Un dispositif de traitement, tel que celui schématisé dans les figures 3 et 4 permet de réaliser une telle neutralisation du formol résiduel présent dans l'atmosphère.

Le dispositif, tel qu'il est schématisé dans la figure 3 est équipé d'un réservoir 16 contenant la solution de neutralisation 17. Il présente par ailleurs des moyens pour réaliser à l'intérieur d'une enceinte 19 un micro-brouillard de la solution de neutralisation.

Le réservoir 16, les moyens de pulvérisation 18 et l'enceinte 19 sont respectivement sensiblement identiques au réservoir 1, aux moyens 8 et au carter principal 5 du générateur d'aérosol. Il faut cependant remarquer que le présent dispositif présente des ouvertures 20 situées à sa périphérie, de préférence juste au-dessus de la surface supérieure de la solution de neutralisation 17 contenue dans le réservoir 16. La dimension globale de ces ouvertures 20 est plus importante que celle de l'ouverture 4, étant donné que dans le cas présent, la réaction a lieu à l'intérieur de l'enceinte 19.

Dans la partie supérieure, le présent dispositif est muni de moyens pour piéger les particules liquides et solides, qui sont constituées par le micro-brouillard de la solution de neutralisation elle-même, et par le produit de la réaction de cette solution sur le formol.

Ces moyens sont constitués par une cheminée 21, qui prolonge de manière continue l'enceinte 19. L'orifice de communication entre l'intérieur de l'enceinte 19 et la cheminée 21 est partiellement obturé par un disque 22 percé en son centre d'un orifice 23. Ce disque 22 présente par ailleurs de préférence, deux rebords circulaires 24 orientés vers l'intérieur de l'enciente 19 et coaxiaux. Le disque 22 constitue un premier piège pour les particules solides et liquides véhiculées par l'atmosphère en sortie de l'enceinte 19.

Par ailleurs, la cheminée 21 est également munie de portions de disques 25 qui s'étendent sensiblement perpendiculairement à l'axe de la cheminée, depuis sa périphérie intérieure vers son axe.

Les portions de disques 25 sont réparties à différents niveaux de la cheminée et disposées les unes par rapport aux autres de manière à obturer au moins une fois l'orifice défini par la cheminée lorsqu'elles sont vues selon son axe 26. Les portions de disques constituent ainsi une succession de chicanes qui retiennent les particules liquides et solides qui sont encore véhiculées par l'atmosphère après son passage au travers de l'ouverture 23.

Les figures 4a à 4h schématisent une disposition préférentielle des portions de disques 25. Ces figures 4a à 4h schématisent la position des huit portions de disques représentées dans la figure 3, successivement depuis la base de la cheminée vers sa partie supérieure 27.

De préférence, les portions de disques présentent un angle au centre sensiblement égal, et par exemple sensiblement égal à 60 degrés. Elles sont regroupées par paires, qui sont constituées par deux portions situées à des niveaux successifs. Pour chaque paire, les portions sont diamétralement opposées.

Ainsi, tel que cela est visible dans les figures 4, les portions 28 et 29 sont diamétralement opposées et il en est de même pour les portions 30 et 31, 32 et 33, 34 et 35.

Par ailleurs, les portions correspondantes de deux paires successives sont décalées de manière à ce que leur bissectrice forme un angle égal sensiblement au double de l'angle au centre des portions. Ainsi, la portion 30 est décalée par rapport à la portion 28, de manière à ce que l'angle de leur bissectrice soit égal au double de leur angle au centre, c'est-à-dire approximativement 120 degrés. Il en est de même pour les portions 29 et 31, 30 et 32, 31 et 33, 32 et 34, 33 et 35.

Par ailleurs, de préférence, la distance selon l'axe 26 de deux portions successives d'une même paire est inférieure à la distance de deux portions successives appartenant à des paires différentes.

D'autre part, dans un mode préférentiel de réalisation, tel que cela est schématisé dans les figures 3 et 4, les portions sont au nombre de huit, regroupées en quatre paires, et les portions de la paire supérieure sont parallèles aux portions correspondantes de la paire inférieure.

En outre, les portions et la cheminée sont réalisées de préférence en un matériau métallique, compatible avec les produits chimiques utilisées.

De manière à favoriser la circulation de l'air à l'intérieur de l'enceinte, qui pénètre par les ouvertures 20 et est évacuée au niveau de l'ouverture 27 de la cheminée 21, une hélice de ventilateur 36, entraînée en rotation par le moteur 37 est de préférence située entre le disque 22 et les portions de disques 25. Par ailleurs, éventuellement, au niveau de la sortie 27 de la cheminée, une hélice de ventilateur 38, entraînée également en rotation par un moteur 39 favorise l'évacuation de l'atmosphère débarrassée du formol, et compense les pertes de charge qu'elle subit à la traversée des chicanes de la cheminée 21. Ce moteur est propre à l'hélice 38.

Ainsi, les deux phases du procédé de désinfection sont respectivement réalisées au moyen des dispositifs représentés dans les figures 1 et 2, 3 et 4. Ces deux dispositifs jouent un rôle complémentaire. De préférence, ils sont regroupés sur un support mobile, et peuvent être ainsi véhiculés d'un local à un autre. D'autre part, des moyens de commande automatiques ou semi-automatiques réalisent leurs fonctionnements respectifs successivement, pendant des durées déterminées, qui sont principalement fonction du volume d'atmosphère à désinfecter.

## Revendications

1. Procédé de désinfection d'un local, tel que par exemple une salle ou un bloc opératoire, dans lequel on pulvérise dans l'atmosphère un micro-brouillard d'une solution d'aldéhyde formique pendant une durée déterminée, caractérisé par le fait qu'ensuite, on provoque le passage de l'atmosphère pendant une durée déterminée, afin d'éliminer le taux résiduel de la solution d'aldéhyde formique présent dans l'atmosphere désinfectée, dans une enceinte à l'intérieur de laquelle on réalise un micro-brouillard d'une solution aqueuse neutralisant l'aldéhyde formique véhicule par l'atmosphère, que l'on sépare au niveau de la sortie de l'enceinte les particules solides et liquides de l'atmosphère gazeuse débarrassée de l'aldéhyde formique.

2. Procédé selon la revendication 1, caractérisé par le fait que l'on utilise comme solution aqueuse neutralisant l'aldéhyde formique une composition contenant du sulfite de sodium en solution aqueuse.

3. Procédé selon la revendication 2, caractérisé par le fait que l'on ajoute à la solution un acide, de manière à maintenir le pH de la solution de neutralisation voisin de 7 au cours de la réaction.

4. Procédé selon la revendication 3, caractérisé par le fait que le dit acide est de l'acide chlorhydrique.

5. Dispositif de traitement d'une atmosphère désinfectée par de l'aldéhyde formique, pour la mise en oeuvre du procédé selon la revendication 1, comprenant une enceinte (19) avec une zone d'entrée (20) pour l'atmosphère à débarrasser de l'aldéhyde formique, et une zone de sortie pour l'atmosphère débarrassée de l'aldéhyde formique, un réservoir (16) contenant une solution aqueuse (17) neutralisant l'aldéhyde formique, des moyens (18) pour générer à l'intérieur de l'enceinte un micro-brouillard de la solution neutralisante (17), et des moyens pour forcer la circulation de l'atmosphère de l'entrée (20) vers la sortie de l'enceinte (19), caractérisé par le fait que la sortie est constituée par une cheminée (21) sensiblement cylindrique de révolution munie d'une pluralité de portions de disques (25) s'étendant depuis la périphérie de la cheminée (21) vers sa zone centrale, sensiblement perpendiculairement à son axe (26), réparties à différents niveaux et disposées de manière à obturer au moins une fois l'orifice de sortie de la cheminée lorsqu'ils sont vus selon son axe (26).

6. Dispositif selon la revendication 5, caractérisé par le fait que les portions de disques (25) présentent un angle au centre sensiblement égal.

7. Dispositif selon l'une quelconque des revendications 5 ou 6, caractérisé par le fait que les portions de disques (25) sont regroupées par paire de portions (28 et 29), (30 et 31), (32 et 33), (34 et 35), superposées et diamétralement opposées.

8. Dispositif selon la revendication 7, caractérisé par le fait que les portions de disques correspondantes (28 et 30), (29 et 31) de deux paires successives (28 et 29), (30 et 31) sont décalées de manière à ce que leur bissectrice forme un angle sensiblement égal au double de l'angle au centre des portions.

9. Dispositif selon l'une quelconque des revendications 5 à 8, caractérisé par le fait qu'il comprend huit portions de disques (28 à 35) présentant respectivement un angle au centre de 60 degrés, regroupées en quatre paires (28 et 29), (30 et 31), (32 et 33), et (34 et 35), les portions correspondantes (28 et 34), (29 et 35) de la première et de la dernière paire étant sensiblement parallèles.

10. Dispositif de traitement d'une atmosphère désinfectée d'un local, selon la revendication 5, comprenant en outre un générateur d'aérosol désinfectant, pulvérisant dans l'atmosphère un micro-brouillard d'aldéhyde formique, permettant ainsi la désinfection préalable du dit local, et regroupé sur un support commun, caractérisé par le fait que des moyens de commande automatique ou semi-automatique réalisent le fonctionnement successif du générateur d'aérosol et du dispositif de traitement pendant une durée déterminée.

**Patentansprüche**

1. Desinfektionsverfahren eines Raumes, wie zum Beispiel ein Operationssaal, bei welchem während einer gewissen Zeit die Atmosphäre mit einer Formaldehydlösung sehr dünn eingenebelt wird, dadurch gekennzeichnet, dass danach, zur Auschaltung des Restgehaltes an Formaldehydlösung in der desinfizierten Atmosphäre, diese Atmosphäre während einer gewissen Zeit in einen Raum überführt wird, wo eine wässerige Lösung, die den Formaldehyd in der Atmosphäre neutralisiert, sehr dünn eingenebel wird und dass am Ausgang des Raumes die festen und flüssigen Partikeln der von Formaldehyd befreiten gasförmigen Atmosphäre abgeschieden werden.

2. Verfahren gemäss dem Patentanspruch 1, dadurch gekennzeichnet, dass als neutralisierende wässerige Lösung Formaldehyd in einer Zusammensetzung mit Natriumsulfit in einer wässerigen Lösung benutzt wird.

3. Verfahren gemäss dem Patentanspruch 2, dadurch gekennzeichnet, dass der Lösung eine Säure beigefügt wird, um den pH-Wert der Neutralisierungslösung während der Reaktion in der Nähe des Wertes 7 zu halten.

4. Verfahren gemäss dem Patentanspruch 3, dadurch gekennzeichnet, dass die betroffene Säure Salzsäure ist.

5. Verfahren zur Behandlung einer mit Formaldehyd desinfizierten Atmosphäre zur Anwendung des Verfahrens gemäss dem Patentanspruch 1 beinhaltendeinem Raum (19) mit einem Eingangsbereich (20) für die vom Formaldehyd zu befreiende Atmosphäre und mit einem Ausgangsbereich für die vom Formaldehyde befreite Atmosphäre, einem Behälter (16) mit einer wässerigen Lösung (17) zur Neutralisierung des Formaldehyds, Vorrichtungen (18) zur Erzeugung eines sehr dünnen Nebels der neutralisierenden lösung (17) im Innern des Raumes und Vorrichtungen zur Verdrängung der Atmosphäre vom Eingang (20) zum Ausgang des Raumes (19), dadurch gekennzeichnet, dass der Ausgang sich aus einem annähernd rotationszylindrischen Kamin (21) mit einer grossen Anzahl an Scheibenabschnitten (25), die vom Randbereich des Kamins (21) bis zum annähernd senkrecht zur Achse (26) gelegenen mittleren Bereich hinreichen, die über verschiedene Ebenen verteilt sind und die so angelegt sind, dass sie im Sichtwinkel der Achse (26) mindestens einmal die Austrittsöffnung des Kamines verschliessen, zusammensetzt.

6. Vorrichtung gemäss dem Patentanspruch 5, dadurch gekennzeichnet, dass die Scheibenabschnitte (25) einen annähernd gleichmässigen Mittelpunktswinkel aufweisen.

7. Vorrichtung gemäss irgendeinem der Patentansprüche 5 bzw 6, dadurch gekennzeichnet, dass die Scheibenabschnitte (25) in gestapelten und gegenüberliegenden Abschnittspaaren (28 und 29), (30 und 31), (32 und 33), (34 und 35) zusammengefasst sind.

8. Vorrichtung gemäss dem Patentanspruch 7, dadurch gekennzeichnet, dass die entsprechenden Scheibenabschnitte (28 und 30), (29 und 31) von zwei aufeinanderfolgenden Paaren (28 und 29), (30 und 31) so versetzt sind, dass deren Halbierende einen Winkel bildet, der annähernd dem doppelten des Mittelpunkswinkels der Abschnitte entspricht.

9. Vorrichtung gemäss irgendeinem der Patentansprüche 5 bis 8, dadurch gekennzeichnet, dass sie acht Scheibenabschnitte (28 bis 35), die jeweils einen Mittelpunktswinkel von 60° bilden und die in vier Paaren (28 und 29), (30 und 31), (32 und 33) und (34 und 35) zusammengefasst sind, umfasst, wobei die entsprechenden Abschnitte (28 und 34), (29 und 35) des ersten und des letzten Paares annähernd parallel sind.

10. Vorrichtung zur Behandlung der desinfizierten Atmosphäre eines Raumes gemäss dem Patentanspruch 5 mit darüber hinaus einem Generator für desinfizierendes Aerosol, der die Atmosphäre sehr dünn mit Formaldehyd einnebelt, wodurch eine vorangehende Desinfektion des entsprechenden Raumes erzielt wird, und der auf einer gemeinsamen Halterung aufgebaut ist, dadurch gekennzeichnet, das die automatischen bzw halb-automatischen Steuerungsvorrichtungen den sukzessiven Betrieb des Aerosolgenerators und der Behandlungsvorrichtung während einer bestimmten Zeit einleiten.

**Claims**

1. A process for disinfecting the premises, such for example as an operating room or block, in which a micro-mist of formaldehyde solution is sprayed into the atmosphere for a given length of time, characterized by the fact that then the atmosphere is caused to pass, for a given length of time, so as to eliminate the residual proportion of formaldehyde solution present in the disinfected atmosphere, through an enclosure inside which a micro-mist is formed of an aqueous solution neutralizing the formaldehyde contained in the atmosphere, and that, at the outlet of the enclosure, the solid and liquid particles are separated from the gaseous atmosphere freed of the formaldehyde.

2. The process according to claim 1, characterized by the fact that a composition containing sodium sulfite in an aqueous solution is used as aqueous solution neutralizing the formaldehyde.

3. The process according to claim 2, characterized by the fact that an acid is added to the solution so as to maintain the pH of the neutralizing solution close to 7 during the reaction.

4. The process according to claim 3, characterized by the fact that said acid is hydrochloric acid.

5. A device for treating an atmosphere disinfected by means of formaldehyde, for implementing the process according to claim 1, comprising an enclosure (19) with an inlet zone (20) for the atmosphere to be freed of the formadehyde, and an outlet zone for the atmosphere freed of the formaldehyde. A reservoir (16) containing an aqueous solution (17) neutralizing the formaldehyde, means (18) for generating inside the enclosure a micro-mist of the neutralizing solution (17), and means for forcing the atmosphere to flow from the inlet (20) towards the outlet of the enclosure (19), characterized by the fact that the outlet is formed by a chimney (21) substantially cylindrical in revolution having a plurality of disk portions (25) extending from the periphery of the chimney (21) to its central zone, substantially perpendicularly to its axis (26), distributed at different levels, and disposed so as to close at least once the outlet orifice of the chimney when they are seen along its axis (26).

6. The device according to claim 5, characterized by the fact that the disk portions (25) present a substantially equal angle at the center.

7. The device according to any one of claims 5 or 6, characterized by the fact that the disk portions (25) are grouped together in pairs of portions (28 and 29), (30 and 31), (32 and 33), (34 and 35), superimposed and diametrially opposite.

8. The device according to claim 7, characterized by the fact that the corresponding disk portions (28 and 30) (29 and 31) of two successive pairs (28 and 29), (30 and 31) are offset so that their bisector forms an angle substantially equal to double the angle at the center of the portions.

9. The device according to any one of claims 5 to 8, characterized by the fact that it comprises eight disk portions (28 and 35) presenting respectively an angle at the center of 60 degrees, grouped together in four pairs (28 and 29), (30 and 31), (32 and 33), and (34 and 35), the corresponding portions (28 and 34), (29 and 35) of the first and of the last pair being substantially parallel.

10. The device for treating a disinfected atmosphere of a premises, according to claim 5, further comprising a disinfecting aerosol generator, spraying into the atmosphere a micro-mist of formaldehyde, for previously disinfecting said premises, and grouped together on a common support, characterized by the fact that automatic or semi-automatic control means provide the successive operation of the aerosol generator and of the treatment device for a given length of time.

Fig 4

Fig 1

Fig 2

Fig 3